# EUROPEAN PATENT APPLICATION

(11) **EP 3 878 357 A1**
(43) Date of publication of application: **15.09.2021**
(21) Application number: 21161803.8
(22) Date of filing: 10.03.2021
(51) Int. Cl.: A61B 5/024, A61B 5/0245, A61B 5/08, A61B 5/00

(54) **WEARABLE ELECTROCARDIOGRAM DEVICES AND METHODS FOR EARLY DETECTION, DIAGNOSTICS AND MONITORING OF INFECTIONS**

(30) Priority: 10.03.2020 US 202062987568 P
(71) Applicant: Vagus Health Ltd., Cambridge CB4 0WS (GB)
(72) Inventor: KRANCK, Gustaf, Cambridge, CB4 0WS (GB)
(74) Representative: Laine IP Oy

(57) **Abstract**

A wrist or finger mounted wearable device and method for determining a state or activity level of the autonomic nervous system, such as the respiratory rate or activity of the vagus nerve. The wearable device having at least a pair of electrocardiograms (ECG) measurement electrode pads configured to perform hand-to-hand ECG measurements. The device or method including monitoring of the pair of ECG measurement pads at a first time period to arrive at a first reading of hand-to-hand ECG, and at a second time period to arrive at a second reading of hand-to-hand ECG. The device or method analyzing the first and second readings of ECG to arrive at first and second indications of a respiratory rate of the individual.

## Description

### BACKGROUND

The vagus nerve is the longest and most complex of the 12 pairs of cranial nerves. It transmits information to and from the brain to organs in the body such as the gut and heart. The vagus nerve is the main component of the parasympathetic nervous system which is also known as the 'rest and digest' system. The 'opposite' of this system is the sympathetic nervous system which is known as the 'fight and flight' system. It is activated when the body is in stress such as during disease progress.

The autonomic nervous system is the main nervous system involved in the body's stress response. The vagus nerve is a very important part of the autonomic nervous system which enables, for instance, relaxation. The vagus nerve is also the most important nerve for immune system responses and it is considered to be very important in the treatment of autoimmune diseases, depression, anxiety and other diseases that require lowering or raising the immune system responses.

Recent advances in immunology reveal a significant pathogenic role for inflammation in the development and progression of inflammations in cardiac- or respiratory systems

In China the Coronavirus prevention during first months were focusing on mitigating the spread of the virus by preventing people from travelling and meeting in large crowds. Healthcare professionals and officials in China and around the world are currently conducting large scale screenings in public spaces, public transport and for instance when crossing borders. There currently exists only three non-invasive traditional risk screening methods for Coronavirus: 1) body temperature 2) outwards signs such as coughing 3) self-reporting of 'feeling sick'. All of these screening methods are detecting the disease only after the person has become sick. When the screening- or healthcare professionals evaluate the risk of infection to be high, the person can be quarantined, CT-scans are done for signs of pneumonia and a blood test is collected for PCR detection.

There is great demand for quick and reliable ways of early detection of diseases for instance in persons suspected of having bacterial or viral respiratory diseases. Further the worldwide onset of COVID-19 has revealed a lack of digital health monitoring and early detection systems of such diseases.

### SUMMARY OF THE INVENTION

The invention is defined by the features of the independent claims. Some specific embodiments are defined in the dependent claims.

According to a first aspect of the present invention, there is provided a wrist or finger mounted wearable device for determining a state or activity level of the autonomic nervous system, such as the respiratory rate or activity of the vagus nerve, the wearable device comprising: at least a pair of electrocardiograms (ECG) measurement electrode pads configured to perform hand-to-hand ECG measurements, at least one processing core and at least one memory including computer program code, the at least one memory and the computer program code being configured to, with the at least one processing core, cause the wearable device to: monitor the pair of ECG measurement pads at a first time period to arrive at a first reading of hand-to-hand ECG, monitor the pair of ECG measurement pads at a second time period to arrive at a second reading of hand-to-hand ECG, and analyze the first and second readings of ECG to arrive at first and second indications of a respiratory rate of the individual.

According to a second aspect of the present invention, there is provided a method of determining a state of health for an individual by monitoring the breathing of an individual via hand-to-hand electrocardiograph, the method comprising the steps of: recording a first phase of the heartbeat of the individual via hand-to-hand electrocardiograph (ECG) during normal breathing, recording a second phase of the heartbeat of the individual via hand-to-hand ECG during controlled or conscious breathing, and comparing the first and second recordings to arrive at an indication of a state of function of the autonomic nervous system of an individual.

### BRIEF DESCRIPTION OF THE DRAWINGS

FIGURE 1 illustrates a smartwatch in accordance with at least some embodiments of the present invention as employed to perform a hand-to-hand ECG test;
FIGURES 2A - 2C show a smartwatch according to certain embodiments of the present invention which prompts a user during performance of a hand-to-hand ECG test via a display of the smartwatch;
FIGURE 3 is an example apparatus capable of supporting at least some embodiments of the present invention;
FIGURE 4 illustrates an example user interface capable of supporting at least some embodiments of the present invention;
FIGURE 5 shows an example breathing and heart rate graph derived from readings during a controlled breathing phase of a test according to certain embodiments of the present invention;
FIGURE 6 shows another example breathing and heart rate graph derived from readings during a controlled breathing phase of a test according to certain embodiments of the present invention;
FIGURE 7 shows a QRST profile of an ECG recording, and
FIGURE 8 shows the vagus nerve and connected systems.

### EMBODIMENTS

### DEFINITIONS

In the present context, the term 'vagal' is used as 'relating to the vagus nerve' .

Vagal tone refers to the level of activity and health of the vagus nerve.

Heart rate variability (HRV) provides for a method to evaluate vagal tone or vagus nerve activity. However, HRV has a major statistically originating flaw when used in short term measurements. Since the heart beats only on average once per second during a resting heart rate - the statistical accuracy when measuring only 20 or 30 heartbeats (25 second test), is on average low. However, methods and devices according to embodiments of the present invention monitor amplitude changes and electrocardiogram's (ECG) QRST-peaks and internal patterns in order to arrive at a metric which can reveal consistent early indicators for the onset of disease.

Certain embodiments of the present invention provide for a Vagus ECG Test as a novel standardized test protocol enabling disease, immune system and vagus nerve measurement and assessment. In recording and analysis of more than 10 000 hand-to-hand ECGs, embodiments of the present invention have been proven to detect changes in the cardiac-respiratory synchronization (RSAsync). RSAsync being a measurement of cardiac/respiratory synchronization. RSAsync patterns can provide for reliable and consistent indication when users have become sick or have mentally derived issues which affect the vagus nerve. Heart rate and breathing are generally considered to be synchronized by the oxygenation effect, whereby heart rate increases at inspiration and decreases at expiration. It has been shown that when the immune or nervous systems in general are disrupted, this synchronization is altered or disappears. These changes in RSAsync can be used for early indicators of disease, infection and continuous monitoring of disease- and for instance medication effects.

The RSAsync can reliably be monitored and measured via embodiments of the present invention which provide for a hand-to-hand ECG test, for example as provided by a watch or ring. Certain embodiments provide for a standardized Vagus ECG Test consisting of at least a period of normal resting ECG measurement followed by a controlled breathing phase where the user is instructed how to conduct regular inhales / exhales each lasting at least 5 seconds. In some embodiments a cloud based analytics system with big data processing is used to analyze data derived from a standardized test, for example in order to provide for an indication of health.

RSAsync may be used within certain embodiments of the present invention to describe and quantify how well the vagus nerve is able to provide optimal oxygenation and blood pressure to the body during breathing and especially during the tests as discussed herein such as the Vagus Test of Vagus ECG test. At least some embodiments employ hand-to-hand ECG in determining an indication of vagal tone, vagal homeostasis and immune system activity levels.

Embodiments of the present invention provide for measurement of a person's breathing capability and relative strength during conscious controlled breathing compared to base breathing, for example, normal breathing or uncontrolled breathing. As one part of at least some embodiments of the present invention, this capability is established and quantified as a 'Vagus Breathing Index' (VBI).

Within some embodiments, a measurement of the heart rate changes as compared to breathing activity, is achieved by using so-called controlled breathing whereby the user consciously inhales and exhales at longer periods than during normal or not consciously influenced breathing. In order to reach optimal dynamic blood pressure and oxygenation, the heart rate should normally start increasing and decreasing before the end of each breath-cycle change.

Once again, one important function of the vagus nerve is that it is synchronizing the heart beats to breathing in order to optimise oxygen delivery to body tissues. This function is more active when the body is relaxed. When the body is stressed from mental issues or disease, this heart/breathing synchronization is altered.

Vagus Tests according to some embodiments of the present invention enable measurement of both base- and controlled breathing and comprise 2 phases. First the user is recording the hand-to-hand ECG with normal base breathing for at least 30 seconds and then the user is consciously affecting breathing so that each inhale and exhale is lasting at least 5 seconds each. This controlled breathing test period should last at least 1 minute. The heart rate variability (HRV) during controlled breathing tests in such systems are compared to the HRV during base breathing phase directly prior to controlled breathing. The relation between these HRV values are part of the indication for the health of the autonomic nervous system (HRVrel).

Described herein are methods and devices to test and calculate RSA, RSAsync, HRVrel, HRmax, HRmin, HR and VBI. These indicators, either separately or in some combination, provide an indication of an individual's autonomic nervous system state and health. These indicators may be used, for example in combination with historical or other data for therapeutic or diagnostic purposes. As an example, the vagus nerve and general health of the user - with a sufficient degree of precision - can be characterized by any combination of: RSA, RSAsync, Pulse, Breathing Index, HRV, HRV change during the Vagus ECG Test, Maximum and Minimum heart beat levels, change of heart activity when comparing base breathing to control breathing, activity levels, sleep, blood pressure change. Embodiments of the present invention provide for methods and devices for calculating each of the above indicators. Certain embodiments provide for an indicator of the vagus nerve, which may be a composite of previous indicators, such an index may be referred to as the 'Vagal Homeostasis Index' (VHI).

Embodiments of the present invention find use in both wellness and medical purposes. When certain embodiments of the invention are sold and used as a wellness device, the analytics and system may be configured such that they only inform the user of the values presented earlier and as an index called 'Vagal Homeostasis' - without making any medical diagnostics or statements. In embodiments of the invention sold and used as a medical device, the analytics and system may make medical diagnostics and advise the user of possible ways and methods to treat the detected disease. In such cases, the device and system may be been approved and classified for medical diagnostics by the appropriate medical device approval authorities.

Heart rate variability (HRV) may serve as a fundamental indicator of vagal tone, for example, the level of activation of the vagus nerve influence on the heart. Measures of HRV have been strongly correlated to morbidity and mortality from diverse diseases.

At least some embodiments of the present invention measure vagal tone based on heart rate variability (HRV as SDNN or rMSSD) and respiratory sinus arrhythmia (RSA) which is a measure of the heart rate's systematic pattern and amplitude as calculated, for example, by using Fourier Transformation (FT) analytics to establish frequency patterns.

At least some embodiments of the present invention may provide an indication of sepsis 12 to 24 hours prior to traditional clinical methods and markers, for example, fever or tachycardia.

Short timespan HRV measurements and tests, for example those lasting less than five minutes, have been shown to be unreliable in certain circumstances. At least some embodiments of the present invention provide for a solution to this issue by combining at least some of: Respiratory Sinus Arrhythmia (RSA), Cardiac/Respiratory Synchronization (RSAsync), heart rate variability (HRV), resting pulse and other bio-signal measurements in order to arrive at a more reliable indicator. For example such values may be derived from wearable devices in order to arrive at better and more comprehensive vagus nerve activity analysis than with traditional HRV analytics.

Immune system changes affect multiple vagus nerve activities and can be measured by performing a Vagus Test as described herein.

During the SARS epidemic in 2003 it was found that people had tachycardia which is an indication that the vagus nerve was under activated and hence the cardiac/breathing synchronization was compromised. Tests according to embodiments of the present invention provide for a reliable indication that the vagus nerve is under activated and thus may provide for early warning and detection of an epidemic.

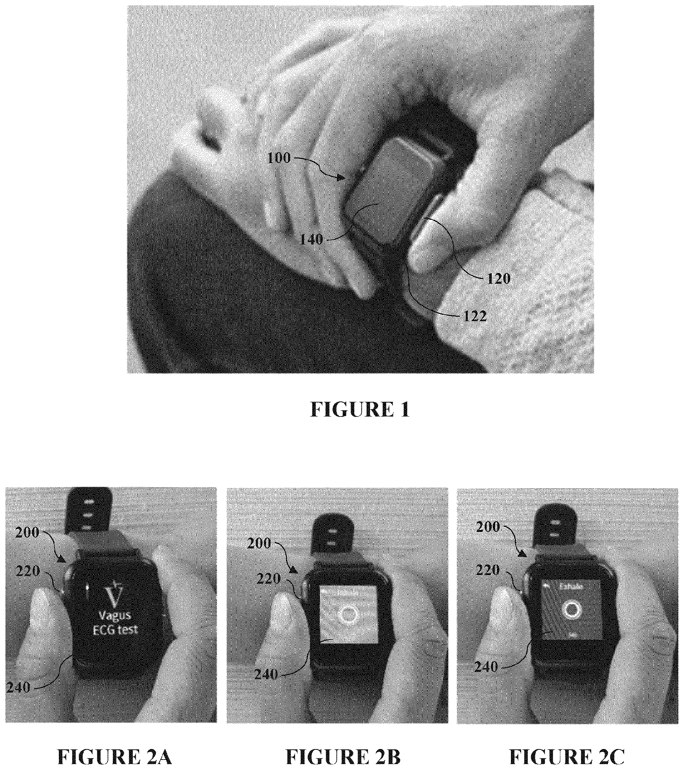

FIGURE 1 illustrates a smartwatch 100 in accordance with at least some embodiments of the present invention as employed to perform a hand-to-hand ECG test. As shown, the a wrist or finger mounted wearable device 100 for determining a state or activity level of the autonomic nervous system, such as the respiratory rate or activity of the vagus nerve is mounted on the wrist of the person and the other hand is brought into contact with a ECG pad 120. The other ECG pad 122 being mounted on the base of the watch. In least some embodiments of the present invention wireless forms of ECG measurement are employed in place of or in addition to the pads, for example, infrared ECG measurement. ECG pads as discussed herein may take a wide variety of forms and at least some embodiments at least partially employ wireless ECG measurement.

As per FIGURE 1, the wearable device 100 comprises at least a pair of electrocardiograms (ECG) measurement electrode pads 120 and 122 configured to perform hand-to-hand ECG measurements. The device 100 also comprises at least one processing core and at least one memory including computer program code not shown as they are comprised within the smartwatch of FIGURE 1. The at least one memory and the computer program code being configured to, with the at least one processing core, cause the wearable device to: monitor the pair of ECG measurement pads 120, 122 at a first time period to arrive at a first reading of hand-to-hand ECG, monitor the pair of ECG measurement pads 120, 122 at a second time period to arrive at a second reading of hand-to-hand ECG, and analyze the first and second readings of ECG to arrive at first and second indications of a respiratory rate of the individual.

Certain embodiments of the present invention comprise a wrist or finger mounted wearable device configured to compare the first and second indications of the respiratory rate of the individual to arrive at an indication of the state or activity level of the vagus nerve.

As also shown in FIGURE 1, at least some embodiments of the present invention provide for a wrist or finger mounted wearable device further comprising a display 140. Such a display may be configured to provide prompts to a user, for example via text or graphics, such as animated graphics.

Certain embodiments of the present invention provide for an indication of breathing or respiratory rate by measuring electrical potential difference directly between hands of a person. In such situations the amplitude of voltage measurements change when the person inhales and exhales.

The reason for these voltage amplitude changes are as follows; during inspiration, there is diaphragmatic contraction in the heart which, because the pericardium is attached to the central tendon of the diaphragm, causes caudal and rotational movement of the heart and vice versa. This rotational movement in turn cause changes in conduction especially in the direction of aVR and aVL electrodes in standard 12-lead ECG measurement. This amplitude change is clearly visible as changes in the R and S amplitude in the hand-to-hand ECG and provides for indications of breathing in at least some embodiments of the present invention.

FIGURES 2A - 2C show a smartwatch 200 according to certain embodiments of the present invention which prompts a user during performance of a hand-to-hand ECG test via a display 240 of the smartwatch while the user places their thumb on an ECG pad or sensor 220 of the smartwatch 200.

As shown in FIGURE 2A the user is informed that a Vagus ECG test has been initiated and is waiting for instruction. The user is then prompted to breathe in a particular fashion by FIGURE 2B instructing the user to inhale for a predetermined time and then to exhale for a predetermined time within FIGURE 2C.

Certain embodiments of the present invention provide for a device which prompts a user to breathe in a predetermined fashion. Certain embodiments prompt a user to breath at a particular rate. Some embodiments provide for a prompted breathing at a frequency of 0.1 Hz. Such prompts may be configured such that breathing is guided during at least one of the first and second time periods. In certain embodiments the first time period is at least 30 seconds and the second time period is at least 1 minute. Some embodiments prompt a user to breath in a predetermined fashion during the second time period, such embodiments may prompt the user to breathe at a predetermined frequency, for example the 0.1 Hz frequency mentioned above. Certain embodiments prompt a user to breathe normally during at least one of the periods, for example the first time period. In at least some embodiments the first time period precedes the second time period. Such time periods may be referred to as hand-to-hand ECG-derived respiration (EDR) periods.

Within some embodiments of the present invention the second time period follows the first time period such that the second time period is within 10 seconds of, preferably 5 seconds of, more preferably immediately after, the first time period.

Certain embodiments of the present invention are configured to prompt a user in coordination with an external device, such as a mobile phone. Some embodiments are configured to coordinate in multiple ways with an external device, such as a mobile phone.

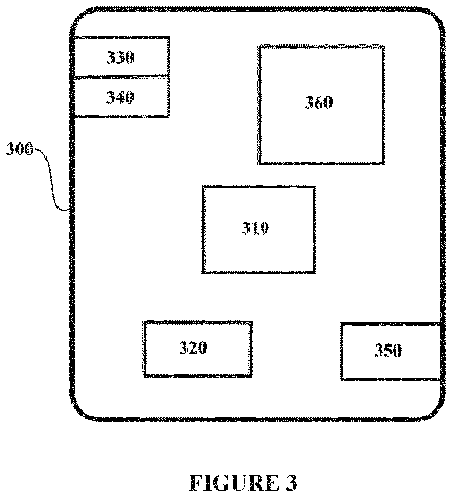

FIGURE 3 illustrates an example apparatus capable of supporting at least some embodiments of the present invention. Illustrated is device 300, which may comprise, for example, a computer device such as controller 150 of FIGURE 1. Comprised in device 300 is processor 310, which may comprise, for example, a single- or multi-core processor wherein a single-core processor comprises one processing core and a multi-core processor comprises more than one processing core. Processor 310 may comprise a Qualcomm Snapdragon 800 processor, for example. Processor 310 may comprise more than one processor. A processing core may comprise, for example, a Cortex-A8 processing core manufactured by Intel Corporation or a Brisbane processing core produced by Advanced Micro Devices Corporation. Processor 310 may comprise at least one application-specific integrated circuit, ASIC. Processor 310 may comprise at least one field-programmable gate array, FPGA. The aforementioned processor types are nonlimiting examples, alternatively an Intel i7 processor, or another suitable type of processor, may be employed.

Device 300 may comprise memory 320. Memory 320 may comprise random-access memory and/or permanent memory. Memory 320 may comprise at least one RAM chip. Memory 320 may comprise magnetic, optical and/or holographic memory. Memory 320 may be at least in part accessible to processor 310. Memory 320 may be means for storing information. Memory 320 may comprise computer instructions that processor 310 is configured to execute. When computer instructions configured to cause processor 310 to perform certain actions are stored in memory 320, and device 300 overall is configured to run under the direction of processor 310 using computer instructions from memory 320, processor 310 and/or its at least one processing core may be considered to be configured to perform said certain actions.

Device 300 may comprise a transmitter 330. Device 300 may comprise a receiver 340. Transmitter 330 and receiver 340 may be configured to transmit and receive, respectively, information in accordance with systems, for example transmitter 330 may transmit information to a monitor for display to a user, and/or receiver 340 may receive input information concerning a location and/or orientation of a further device.

Device 300 may comprise a near-field communication, NFC, transceiver 350. NFC transceiver 350 may support at least one NFC technology, such as NFC, Bluetooth, Wibree or similar technologies.

Device 300 may comprise user interface, UI, 360. UI 360 may comprise at least one of a display, a keyboard and a touchscreen. A user may be able to operate device 300 via UI 360, for example to start or terminate execution of programs.

Processor 310 may be furnished with a transmitter arranged to output information from processor 310, via electric leads internal to device 300, to other devices comprised in device 300. Such a transmitter may comprise a serial bus transmitter arranged to, for example, output information via at least one electric lead to memory 320 for storage therein. Alternatively, to a serial bus, the transmitter may comprise a parallel bus transmitter. Likewise, processor 310 may comprise a receiver arranged to receive information in processor 310, via electrical leads internal to device 300, from other devices comprised in device 300. Such a receiver may comprise a serial bus receiver arranged to, for example, receive information via at least one electric lead from receiver 340 for processing in processor 310. Alternatively, to a serial bus, the receiver may comprise a parallel bus receiver.

Device 300 may comprise further devices not illustrated in FIGURE 3. For example, where device 300 comprises a computer device, it may comprise at least one clock or auxiliary power unit, APU to provide battery power in case of mains power failure.

Processor 310, memory 320, transmitter 330, receiver 340, NFC transceiver 350 and/or UI 360 may be interconnected by electric leads internal to device 300 in a multitude of different ways. For example, each of the aforementioned devices may be separately connected to a master bus internal to device 300, to allow for the devices to exchange information. However, as the skilled person will appreciate, this is only one example and depending on the embodiment various ways of interconnecting at least two of the aforementioned devices may be selected without departing from the scope of the present invention.

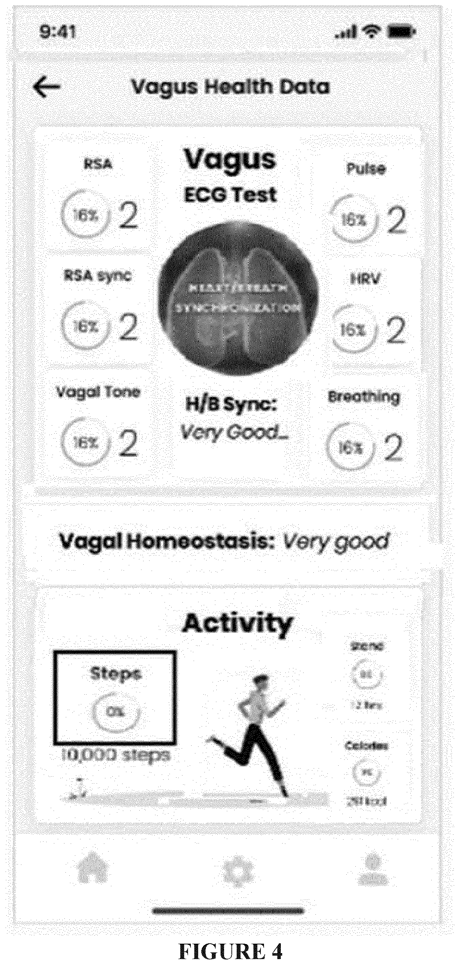

FIGURE 4 illustrates an example user interface capable of supporting at least some embodiments of the present invention, displaying a user's health changes and disease risk assessments.

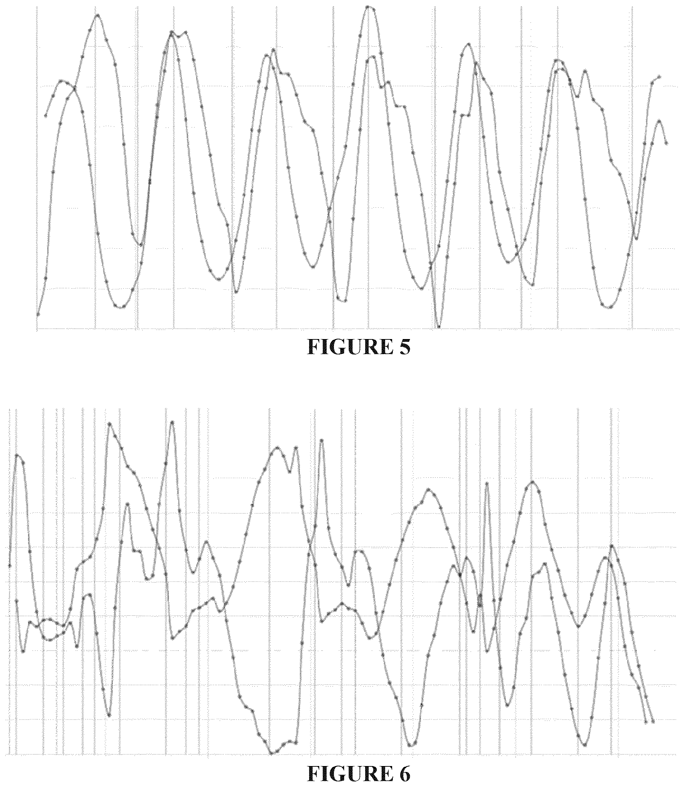

FIGURE 5 shows an example breathing and heart rate graph derived from readings during a controlled breathing phase of a test according to certain embodiments of the present invention. Within the graph, the line which begins at the bottom left of the graph represents a measured or calculated breathing of the person performing the test with the other line representing the heart rate. Within FIGURE 4 the vagal homeostasis and RSAsync is good. As seen the two lines are rather synchronized.

FIGURE 6 shows another example breathing and heart rate graph derived from readings during a controlled breathing phase of a test according to certain embodiments of the present invention. This time, the line which begins below the other line of the graph represents a measured or calculated the heart rate and the other line represents breathing of the person performing the test. FIGURE 5 shows a low vagal homeostasis and RSAsync, indicating that the person is sick, for example sick with a chronic respiratory infection.

At least some embodiments of the present invention provide for a method of determining a state of health for an individual by monitoring the breathing of an individual via hand-to-hand electrocardiograph, the method comprising the steps of: a first phase of recording the heartbeat of the individual via hand-to-hand electrocardiograph (ECG) during normal breathing, a second phase of recording the heartbeat of the individual via hand-to-hand ECG during controlled or conscious breathing, comparing the first and second recordings to arrive at an indication of a state of function of the autonomic nervous system of an individual.

Certain embodiments of the present invention provide for a method wherein a statistical analysis of the breathing data is performed in order to arrive at an index representative of state of health, such as a breathing index, vagus index. Some embodiments providing for a method wherein the normal breathing data and controlled or conscious breathing data is compared to each other in order to arrive at an index indicative of the state of health of the autonomous nervous system, such as a breathing index, vagus index, or vagus breathing index. Certain embodiments providing a method wherein the statistical analysis is at least one of: a percent variation and a percent of average analysis.

At least some embodiments of the present invention determine the overall amplitude of breathing from the hand-to-hand ECG.

Embodiments of the present invention provide for uses in a wide variety of applications. For example:
1) Detecting the degree of oxygenation
2) Detecting the efficiency, volume and periodicity of breathing
3) Detecting dynamic blood pressure
4) Diagnosing and evaluating changes in the user's stress levels
5) Diagnosing the state and activity level of the user's immune system
6) Diagnosing and evaluating changes in inflammation levels
7) Diagnosing and evaluating changes in the user's fatigue and chronic fatigue
8) Diagnosing and evaluating changes in the user's autoimmune diseases
9) Diagnosing and evaluating changes in the user's gut diseases such as IBD and Crohns disease
10) Screening for psychiatric diseases
11) Early detection and evaluating changes in the user's asthma attacks
12) Early detection and evaluating changes in the user's anxiety attacks
13) Early detection and evaluating changes in the user's rage outbursts
14) Early detection and evaluating changes in the user's fear response
15) Evaluation of the user's autonomic nervous system reactions to different types of music
16) Evaluation of the effects from alcohol-, drug- or other types of abuses
17) Detection of harmful sinus arrhythmias
18) Providing neurofeedback for vagus nerve stimulation and neuromodulation devices and systems
19) Fit for duty tests of professional pilots and ship captains, for example ensuring they have received the required amount of rest or are not in an altered state.
20) Evaluation of the user's health and fitness levels for sports and training purposes.

At least some systems according to certain embodiments of the present invention compromise some of the following features:
a) Signal transfer to and from the device with Wireless Bluetooth, Zigbee or other radio transmission to either a smartphone or directly to the data cloud by wireless data transmission.
b) Signal transfer, processing and feedback in a smartphone and its dedicated app.
c) Cloud processing
d) At least some embodiments also comprise:
   a. Individualization
   b. Neurofeedback from non-invasive or invasive vagus- or other neuro stimulation
   c. Feedback from autonomic nervous system interventions with pharmacology, neurostimulation or health/wellness treatments.

Disease monitoring and detection systems according to some embodiments of the present invention compromise at least the following analytical methods where the system is combining parameters to a standardized evaluation of changes to the persons immune response and hence probability for that the person to have contracted a disease:
a) The here presented novel Respiratory Sinus Arrhythmia Synchronization (RSAsync) calculation consisting of estimating each breath corresponding heart rate pattern and timing and establishing an index (RSAsync) which is expressing the degree of normality for this synchronization.
b) The heart rate QRST pattern and amplitude so that for instance amplitude decrease and/or T-wave amplitude and placement increase represent

In addition to above analytical methods the certain systems according to the present invention also include at least one of the following parameters when establishing changes to the persons immune system and probability to have contracted a disease:
a) Heart Rate Variability (HRV) as calculated from ECG- and optical pulse measurements
b) Pulse as measured both with ECG and the watch/ring optical pulse sensor
c) Blood Pressure as measured with the watch/ring optical pulse and blood pressure measurement algorithms
d) Electrodermal activity as measured with the watch/ring galvanic skin response sensor
e) Pulse pattern changes between the normal breathing and controlled breathing phase in the Vagus ECG Test.
f) Personalization with artificial intelligence systems based on the person's previous data and all users general data.

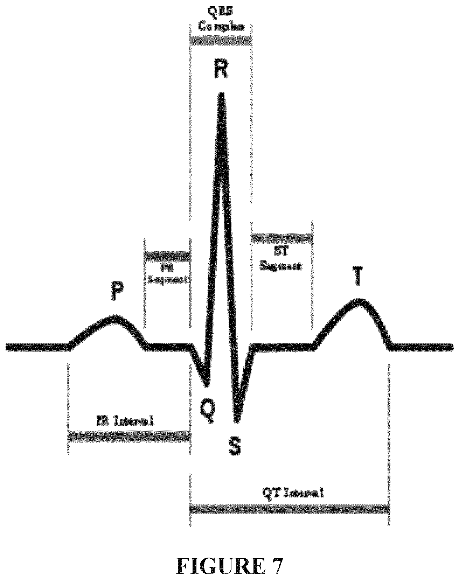

FIGURE 7 shows an example QRST profile of an ECG recording as may be derived by at least some embodiments of the present invention.

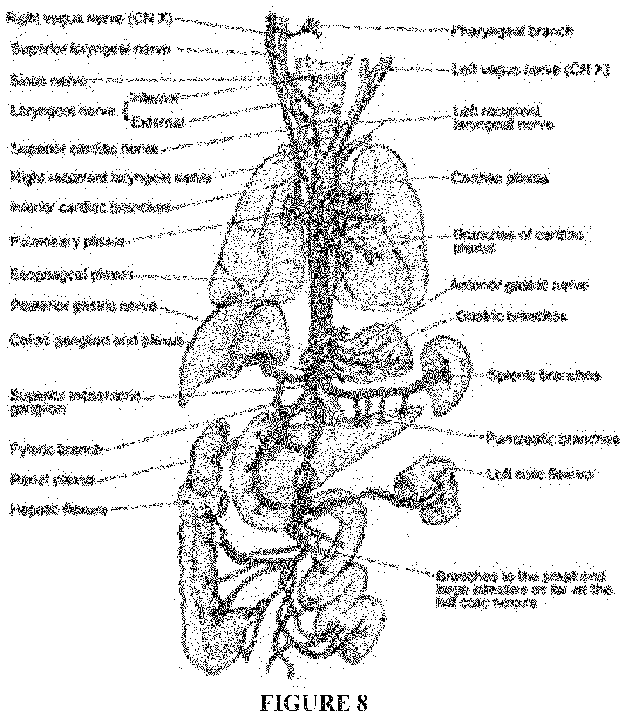

FIGURE 8 shows the vagus nerve and connected systems.

At least some systems according to certain embodiments of the present invention comprise at least one component from the following:
A) A wearable ECG device in the form of a single lead electrocardiogram enabled smartwatch or -ring. This wearable ECG device can transfer measured data to a smartphone or direct to the cloud through wireless transfer such as WIFI or SIM-card enabled data transfer. If the device cannot communicate directly wirelessly with the below mentioned CDPSU, then the system may employ the third part mentioned in c)
B) A Central Data Processing and Data Storage Unit (CDPSU) either as cloud processing or other solution. This cloud processing enable the analytics of all collected data, storage of historical data and distribution of data to third parties such as for instance doctors or other 3^{rd} parties. This central part of the system also contains so-called bots which together with artificial intelligence can advise and help the user to better manage his/her health and disease.
C) A smartphone, pad, computer or other type of data transfer and user interface display device with an application software which is receiving the data from the wearable ECG device, storing the data, displaying the data and analysis results, transferring and receiving data and analytics results from the central data processing and storage unit.

After performing a test, the wearable ECG device (A) according to at least some embodiments of the present invention transmits the data to the transfer and display device which then transmits the data to the CDPSU for analysis. The analytics results are transmitted back to the phone/device (A) and (C) within a short time period - normally a few seconds. At least some Vagus Tests according to the present invention are such that disease assessment is done in the cloud and it is shown on a local device, such as a phone app within seconds from finishing the test.

At least some embodiments of the present invention provide for measurement of an ECG signal at 250 Hz signal measurement frequency. Certain embodiments measuring from 250 Hz up to 1000Hz.

Hand-to-hand ECG signals as measured by embodiments of the present invention have many similarities to the signals from 12-lead electrode placements called aVR and aVL. It is estimated that by 2022 there will be 200 million hand-to-hand ECG capable smart watches and fitness bands. Hand-to-hand ECG will hence become the 2nd most common home health diagnostics device after the thermometer.

At least some embodiments of the present invention provide for crowd sourced data collection and analysis with the option for open data access. For example through data anonymization.

Some embodiments of the present invention take the form of a watch/ring device which is waterproof so it can easily be disinfected between uses, for example if used for multiple users. Certain devices can be configured such that a user name or other unique identifier is attached to data from said user in order to provide for multiple user monitoring.

Certain embodiments of the present invention incorporate the option for medical consulting and health coaching. At least some systems enable automatic or separately instructed sharing of data, measurements or analytics results to the user's defined medical professional or other party.

The here presented infection and/or other disease risk assessment combined with traditional screening methods such as measuring temperature or observing external signs such as coughing, provides a better and more comprehensive system than what is now used worldwide.

At least some embodiments of the present invention provide for at least one of the following clauses:
Claus 1: determine at least one of a level and state of 'vagal homeostasis'.
Claus 2: determine if the user's immune system has changed from the time of previous measurements and analytics results.
Claus 3: establish a standardized test protocol called 'Vagus ECG Test', consisting of 30 seconds base breathing and then a 60 second controlled breathing period with 5 seconds inhale and 5 seconds exhale in each breathing cycle.
Claus 4: employ a novel and informative breathing analytics parameter which is called Vagal Breathing Index (VBI) as measurement of the breathing when calculated from the QRS amplitude changes during the controlled 60 second breathing phase of the 'Vagus ECG Test'.
Claus 5: employ a novel and informative analytics parameter which is called Respiratory Sinus Arrhythmia Synchronization (RSAsync) as the measurement of the cardiac/respiratory synchronization. This value is at least partially derived from the ECG measurement obtained during the 'Vagus ECG Test'.
Claus 6: calculate a Vagal Homeostasis Index (VHI), for example, a VHI which is between 1-100 where 100 is considered as excellent vagal homeostasis and 1 is very bad or very low level of vagal homeostasis.
Claus 7: determine a five level homeostasis rating which is an indicator of the measured persons homeostasis level and immune system activity levels and they are presented to the user as a) very high, b) high, c) moderate, d) low or e) very low or other suitable wordings or sound expressions from the devices.

In at least some embodiments the Vagal Homeostasis Index (VHI) and the disease risk rating system is established as an individualized combination of at least the following system measured values: Respiratory Sinus Arrhythmia Synchronization (RSAsync), Heart rate QRST peak pattern and amplitude and Heart Rate Variability (HRV)

In addition to using the previously stated values for establishing the disease risk rating - certain system according to the present invention use one or more of the following values: Respiratory sinus arrhythmia (RSA), Blood Pressure (BP) as measured with the device sensor, respiratory sinus arrhythmia synchronization (RSAsync), the heart rate QRST peak pattern and amplitude, heart rate Variability (HRV), Heart Rate (HR) as calculated either from the ECG or optical heart rate sensors, maximum heart rate during the Vagus ECG Test (HRmax), minimum Heart Rate during the Vagus ECG Test (HRmin), electrodermal activity as measured by a device galvanic skin response sensor, the strength and pattern of breathing as expressed with the inventions Vagus Breathing Index (VBI) and calculated from the Vagus ECG test, artificial intelligence systems based pattern recognition of the ECG patterns during the Vagus ECG Test and other bio signals measured by the wearable ECG watch sensor such as longer term optical heart rate variation, blood pressure, blood oxygenation, activity and sleep.

Immune system activity levels and risk assessments are uniquely determined from the Vagal Homeostasis estimation and individualized additional combinations of the following: Respiratory Sinus Arrhythmia RSA), Respiratory Sinus Arrhythmia Synchronization (RSAsync), Heart Rate Variability (HRV), Heart Rate (HR), Maximum Heart Rate during the Vagus ECG Test (HRmax), minimum Heart Rate during the Vagus ECG Test (HRmin), Vagus Breathing Index as calculated from the Vagus ECG test (VBI), pattern recognition of the ECG patterns during the Vagus ECG Test, other bio signals measured by the wearable ECG watch sensor such as longer term optical heart rate variation, blood pressure, blood oxygenation, activity, sleep, self-reported historical information and artificial intelligence evaluations of the persons individual immune system profile and its change-patterns.

At least some systems use historical data and feedback from the user together with artificial intelligence and self-learning algorithms to refine and improve the analytical values, diagnostics results, conclusions and healthcare advices to improve the person's immune system activity levels and vagal homeostasis.

Devices and methods according to embodiments of the present invention provide for a novel use of a concept of vagal homeostasis to analyze and diagnose a variety of health issues and medical conditions such as stress, anxiety, depression, autoimmune diseases, cancerous diseases, neurological disorders and immune system disorders. The devices and methods can determine from the immune system activity levels if the person has been infected by a bacterial or viral respiratory disease before the person has visible signs of the disease such as fever or coughing.

Embodiments of the present invention provide a method to screen larger groups of people for respiratory or other types of bacterial and viral diseases before the screened person has visible signs of any disease. The results can be presented as a 'disease warning' when vagal homeostasis, immune levels and analytics results are typical for the said disease and person type. This screening process also take into consideration such as age, sex, length and weight. The tested persons screening results is presented wirelessly to the measuring person and the relevant on- or off-site authority monitoring of the screening.

Disease risk-assessment and screening process devices and methods according to some embodiments of the present invention can additionally use a device camera for detection parameters such as skin color, temperature and other visible features detectable with artificial intelligence together with camera vision. Such risk-assessment can be used for critical work tasks and jobs as a screening method to also to detect other types of homeostasis imbalances and immune stress.

Embodiments of the present invention can provide for indication of mental stress levels, depression, anxiety, chronic neurological low-grade inflammations or digestive and gut imbalances. Certain embodiments can detect and evaluate the degree and level of the tested persons 'Post Traumatic Stress Disorder (PTSD).

At least some embodiments of the present invention provide a new diagnostics and analytics method with user feedback capability for evaluating the effect of pharmaceutical medication dosing and it provides a means to establish personalized medication. With some embodiments providing a new analytics and evaluation method for pharmaceutical medication trials.

Some embodiments of the present invention provide feedback for personalized pharmaceutical medication. At least certain embodiments provide a new type of analytics and evaluation models for Alzheimer and dementia when they occur as a result of neurological inflammations.

Devices and systems according to embodiments of the present invention enable long-term health tracking, longer-term data collection and big data machine learning and artificial methods for the system to can detect if the person is becoming sick or if the low vagal homeostasis is due to mental issues, neurological low-grade inflammation or issues connected with the gut and digestions.

With personalized long-term health tracking, longer-term data collection and big data machine learning and artificial methods systems according to the present invention can detect if the person's long-term changes in vagal homeostasis is most likely to occur due to cancer-like tumors.

Analytics systems according to some embodiments of the present invention comprise machine learning algorithms which can improve the differentiation between these previously mentioned causes of 'vagal homeostasis' changes.

Risk-assessments according to certain embodiments of the present invention can be used to detect high stress, high degrees of depression, potential for suicidal thoughts and/or other mental states which seriously can harm the tested person's life or work capability.

Embodiments of the present invention can be used for cardiac-respiratory viral diseases such as the Coronavirus COVID-19 and provide for an improvement over previous non-invasive early-stage disease detection methods

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

## Claims

1. A wrist or finger mounted wearable device (100) for determining a state or activity level of the autonomic nervous system, such as the respiratory rate or activity of the vagus nerve, the wearable device comprising:
- at least a pair of electrocardiograms (ECG) measurement electrode pads (120, 122) configured to perform hand-to-hand ECG measurements,
- at least one processing core and at least one memory including computer program code, the at least one memory and the computer program code being configured to, with the at least one processing core, cause the wearable device to:
- monitor the pair of ECG measurement pads (120, 122) at a first time period to arrive at a first reading of hand-to-hand ECG,
- monitor the pair of ECG measurement pads (120, 122) at a second time period to arrive at a second reading of hand-to-hand ECG, and
- analyze the first and second readings of ECG to arrive at first and second indications of a respiratory rate of the individual.

2. The wrist or finger mounted wearable device (100) of claim 2, wherein the device is further configured to compare the first and second indications of the respiratory rate of the individual to arrive at an indication of the state or activity level of the vagus nerve.

3. The wrist or finger mounted wearable device of any preceding claim, wherein the device is further configured to prompt a user to breathe in a predetermine fashion, such as instructing the user to breathe at a particular rate, in particular to breathe at a frequency of 0.1 Hz.

4. The wrist or finger mounted wearable device of any preceding claim, wherein the first time period is at least 30 seconds and the second time period is at least 1 minute.

5. The wrist or finger mounted wearable device of any preceding claim, wherein the device further comprises a display (140) which displays prompts to the user.

6. The wrist or finger mounted wearable device of any preceding claim, wherein the device is further configured to prompt a user to breathe in a predetermined fashion during the second time period.

7. The wrist or finger mounted wearable device of any preceding claim, wherein the device is further configured to prompt a user to breathe normally during the first time period.

8. The wrist or finger mounted wearable device of any preceding claim, wherein the second time period follows the first time period, the second time period being within 10 seconds of, preferably 5 seconds of, more preferably immediately after, the first time period.

9. The wrist or finger mounted wearable device of any preceding claim, wherein the device is configured to prompt a user in coordination with an external device, such as a mobile phone.

10. A method of determining a state of health for an individual by monitoring the breathing of an individual via hand-to-hand electrocardiograph, the method comprising the steps of:
- a first phase of recording the heartbeat of the individual via hand-to-hand electrocardiograph (ECG) during normal breathing,
- a second phase of recording the heartbeat of the individual via hand-to-hand ECG during controlled or conscious breathing, and
- comparing the first and second recordings to arrive at an indication of a state of function of the autonomic nervous system of an individual.

11. The method of claim 10 wherein a statistical analysis of the breathing data is performed in order to arrive at an index representative of state of health, such as a breathing index or vagus index.

12. The method of claim 10 or 11 wherein the normal breathing data and controlled or conscious breathing data is compared in order to arrive at an index indicative of the state of health of the autonomous nervous system, such as a breathing index, vagus index, or vagus breathing index.

13. The method of claim 11 wherein the statistical analysis is at least one of: a percent variation and a percent of average analysis.

14. The method of any of claims 10 - 13 wherein the overall amplitude of breathing is determined from the hand-to-hand ECG.

15. A non-transitory computer readable medium having stored thereon a set of computer readable instructions that, when executed by at least one processor, cause an apparatus to at least perform the method of any of claims 10 - 14.
